# EUROPEAN PATENT APPLICATION

(11) **EP 3 339 335 A1**
(43) Date of publication of application: **27.06.2018**
(21) Application number: 16206313.5
(22) Date of filing: 22.12.2016
(51) Int. Cl.: C08F 8/30, A61K 6/083

(54) **PROCESS FOR PREPARING A DENTAL RESIN-MODIFIED GLASS IONOMER COMPOSITION**

(71) Applicant: Dentsply DeTrey GmbH, 78467 Konstanz (DE)
(72) Inventor: Scheufler, Christian, 78234 Engen (DE); Renn, Caroline, 78224 Singen (DE); Maier, Maximilian, 78462 Konstanz (DE); Klee, Joachim E., 78315 Radolfzell (DE)
(74) Representative: Blodig, Wolfgang

(57) **Abstract**

The present invention relates to a process for the preparation of a dental resin-modified glass ionomer composition comprising a polymerizable polyacidic polymer obtained by modification of polyacidic polymers with polymerizable compounds containing an amino group and/or an isocyanato group.

## Description

### Field of the Invention

The present invention relates to a process for the preparation of a dental resin-modified glass ionomer composition. The present invention also relates to a dental resin-modified glass ionomer composition obtainable by the process of the present invention.

According to the present invention, a polymerizable linear polyacidic acrylic polymer having polymerizable pendant groups linked to the acrylic polymer backbone by amide groups may be conveniently and efficiently obtained at a high molecular weight for use in a dental resin-modified glass ionomer composition. The polymerizable linear polyacidic acrylic polymer is resistant to acidic media and capable of further crosslinking, providing improved storage stability and long-term mechanical resistance of the dental resin-modified glass ionomer composition.

### Background to the Invention

Dental restorative materials are known for restoring the function, morphology and integrity of dental structures damaged by physical damage or caries-related decay of enamel and/or dentin. Dental restorative materials are required to have high biocompatibility, good mechanical properties and mechanical and chemical resistance over a long period of time given the harsh conditions for a restorative material in the buccal cavity.

Dental restorative materials include dental resin-modified glass ionomer compositions having good biocompatibility and good adhesion to the dental hard tissues. Moreover, dental resin-modified glass ionomer compositions may provide cariostatic properties through the release of fluoride ions. Dental resin-modified glass ionomer compositions are cured by an acid-base reaction between a reactive glass powder and a polyalkenoic acid. However, conventional dental resin-modified glass ionomer compositions have a low flexural strength and are brittle due to salt-like structures between the polyacid and the basic glass.

The mechanical properties of dental resin-modified glass ionomer compositions may be improved by the selection of the polyacidic polymer. For example, a polymer having polymerizable moieties as pendant groups can be crosslinked in order to increase the mechanical resistance of the resulting glass ionomer cement.

Japanese Patent Publication No. 2005-65902A discloses a dental adhesive composition comprising, as a polymerizable monomer containing a particular carboxylic acid, a carboxylic acid compound having a (meth)acryloyl group and a carboxyl group which are bound to an aromatic group. However, such a polymerizable monomer having an ester group quickly degrades in an acidic medium.

Chen *et al.* and Nesterova *et al.* (Chen et al., J. Appl. Polym. Sci., 109 (2008) 2802-2807; Nesterova et al., Russian Journal of Applied Chemistry, 82 (2009) 618-621) disclose copolymers of N-vinylformamide with acrylic acid and/or methacrylic acid, respectively. However, none of these documents mentions the introduction of a further polymerizable moiety into the copolymer.

WO2003/011232 discloses water-based medical and dental glass ionomer cements that can be post-polymerized after the cement reaction. The dental glass ionomer cements consist of two separate polymers, wherein one of the polymers has a pendant post-polymerizable moiety linked to the polymer through an ester bond. However, ester bonds between the polymer and the polymerizable moieties are prone to hydrolytic cleavage in acidic media.

WO2012/084206 A1 discloses a polymer for a process for producing a water-soluble, hydrolysis-stable, polymerizable polymer, comprising a) a step of copolymerizing a mixture comprising (i) a first copolymerizable monomer comprising at least one optionally protected carboxylic acid group and a first polymerizable organic moiety, and (ii) a second copolymerizable monomer comprising one or more optionally protected primary and/or secondary amino groups and a second polymerizable organic moiety, for obtaining an amino group containing copolymer; b) a step of coupling to the amino group containing copolymer a compound having a polymerizable moiety and a functional group reactive with an amino group of repeating units derived from the second copolymerizable monomer in the amino group containing copolymer obtained in the first step wherein the optionally protected amino group is deprotected, so that polymerizable pendant groups are linked to the backbone by hydrolysis-stable linking groups, and, optionally, a step of deprotecting the protected carboxylic acid group after step (a) or step (b), for obtaining a polymerizable polymer.

The preparation of the polymerizable polymer according to WO2012/084206 A1 may be hindered by a salt reaction of a carboxylic acid and an amine base during polymerization unless a protecting group for the first and/or second copolymerizable monomer is used. Specifically, an acid-base reaction may occur during the step of copolymerizing a mixture comprising (i) a first copolymerizable monomer comprising at least one carboxylic acid group, and (ii) a second copolymerizable monomer comprising one or more primary and/or secondary amino groups for obtaining an amino group containing copolymer. The acid-base reaction may form salts having a solubility in the reaction medium which is different from the solubilities of the free acid and base. On the other hand, the use of a protecting group may have an influence on the structure and properties of the polymerizable polymer, and requires an additional deprotection step in order to liberate a maximum number of carboxylic acid groups. Moreover, given that the second copolymerizable monomer does not contain acidic groups, the number of carboxylic groups which may take part in a cement reaction is reduced as the amount of the polymerizable groups is increased in the polymerizable polymer is increased.

### Summary of the Invention

It is the problem of the present invention to provide a process for the preparation of a dental resin-modified glass ionomer composition providing improved mechanical properties including high flexural strength and a clinically relevant adhesion to tooth structure after curing, as well as hydrolysis-stability in an aqueous medium before and after curing, in particular in an acidic medium.

This problem is solved according to the present invention by a process for the preparation of a dental resin-modified glass ionomer composition comprising a polymerizable polyacidic polymer, wherein the process comprises the following steps:
(a) providing a linear polyacidic acrylic polymer having repeating units of the following formula (I) 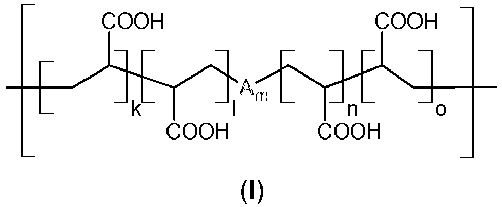 wherein
   A , which may be the same or different, independently is selected from a group of the following formula (Ia) to (If): 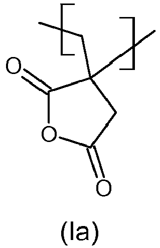 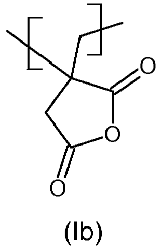 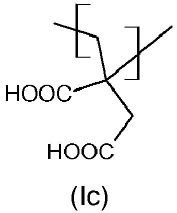 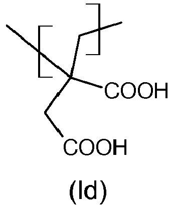 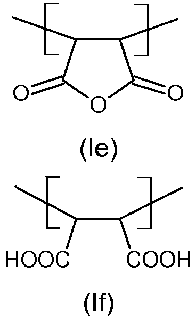
   k, l, m, n and o are independently integers of at least 0,
   k+l+m+n+o is at least 2; and
   at least one of k, l, n, and o is at least 1;
   wherein the polyacidic polymer has a weight average molecular weight of 10 to 300 kDa;
(b) reacting the linear polyacidic acrylic polymer with one or more polymerizable compounds of the following formula (II) in a solvent:

   R-X (II)

   wherein
   X is selected from an amino group and an isocyanato group; and
   R is an organic group having one or more polymerizable double bonds;
   for preparing a polymerizable linear polyacidic acrylic polymer having polymerizable pendant groups linked to the acrylic polymer backbone by amide groups; and
(c) combining the polymerizable linear polyacidic acrylic polymer having polymerizable pendant groups linked to the acrylic polymer backbone by amide groups with a reactive dental glass capable of reacting with the polyacidic acrylic polymer in a cement reaction.

The present invention also provides a dental resin-modified glass ionomer composition obtainable by the process of the present invention.

A cured aqueous dental glass ionomer composition according to the present invention is hydrolysis-stable and has excellent mechanical properties based on the specific combination of the polymerizable polymer.

The process of the present invention provides a polymer useful in an aqueous dental glass ionomer composition, which is hydrolysis-stable and can be polymerized to yield a cured dental glass ionomer composition of improved mechanical resistance. The polymer may be provided with a high amount of acidic groups resulting in an excellent adhesion to dental hard tissue. Moreover, since the process of the present invention provides a polymer having a high molecular weight, any polymer shrinkage during the curing reaction may be easily controlled.

Moreover, the preparation of the polymerizable polymer according to the present invention is not impaired by a salt reaction of a carboxylic acid and an amine base during polymerization. Finally, the use of a protecting group is not required whereby an additional deprotection step is not required. Moreover, the number of the polymerizable groups does not have an influence on the number of carboxylic acid groups so that a maximum number of carboxylic acid groups may be introduced into the polymerizable polymer.

The present inventors have recognized that resin reinforced dental glass ionomer cements are subject to deterioration during storage or after curing in the mouth of the patient. The present inventors have further recognized that the deterioration is due to hydrolytic degradation of the resin component conventionally containing hydrolyzable moieties. The present inventors have then recognized that by using a specific process for the preparation of a polymer, an improved water-soluble, hydrolysis-stable, polymerizable polymer may be prepared at a high molecular weight which overcomes the drawbacks of conventional resin reinforced glass ionomer cements known from the prior art.

The polymerizable pendant groups of the polymerizable polymer may react with a further polymerizable pendant group or a monomer having a polymerizable double bond whereby a crosslinked network or graft polymer is formed.

### Detailed description of the preferred embodiments

The process for the preparation of a dental resin-modified glass ionomer composition according to the present invention comprises for obtaining a polymerizable polyacidic polymer, a step a), a step b), and a step c).

A "polymerizable linear polyacidic acrylic polymer having polymerizable pendant groups linked to the acrylic polymer backbone by amide groups " used in the present invention is a polymerizable polyacidic polymer containing one or more polymerizable moieties allowing polymerization and crosslinking of the polymer after the formation of a glass ionomer cement, increasing the long-term mechanical resistance of the material. The "polymerizable linear polyacidic acrylic polymer having polymerizable pendant groups linked to the acrylic polymer backbone by amide groups" contained used in the present invention is a polymerizable polyacidic polymer is at the same time a polymerizable polyacidic polymer comprising carboxylic acid groups. The carboxylic acid groups of the polymer can react with a reactive glass component to form a glass ionomer cement which can be used as a dental material. The polymerizable linear polyacidic acrylic polymer is water-soluble. The term "water-soluble" means that at least 0.1 g, preferably 0.5 g of the polymer dissolves in 100 g of water at 20°C. The polymerizable linear polyacidic acrylic polymer is hydrolysis-stable. "Hydrolysis-stable" means that the polymer is stable to hydrolysis in an acidic medium, such as in a dental composition. Specifically, the polymer does not contain groups such as ester groups which hydrolyze in aqueous media at pH3 at room temperature within one month.

The present invention provides a process for the preparation of a dental resin-modified glass ionomer composition comprising a specific polymerizable polyacidic polymer.

The process according to the present invention comprises a step (a) of providing a linear polyacidic acrylic polymer having repeating units of the following formula (I) 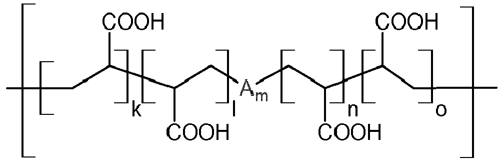 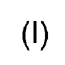 wherein
A , which may be the same or different, independently is selected from a group of the following formula (Ia) to (If): 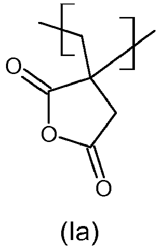 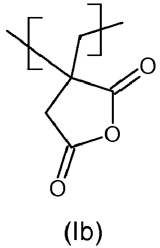 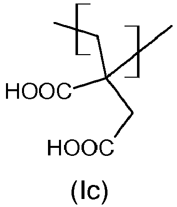 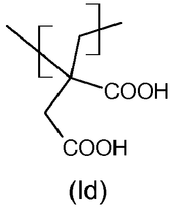 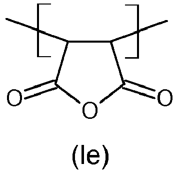 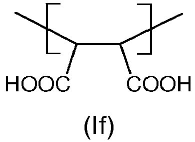
k, l, m, n and o are independently integers of at least 0,
k+l+m+n+o is at least 2; and
at least one of k, l, n, and o is at least 1;
wherein the polyacidic polymer has a weight average molecular weight of 10 to 300 kDa.

A linear polyacidic acrylic polymer of the formula (I) may be prepared based by polymerizing acrylic acid or a mixture comprising acrylic acid.

A mixture comprising acrylic acid may further comprise one or more unsaturated monocarboxylic acids or unsaturated dicarboxylic acids or an anhydride of the unsaturated dicarboxylic acids. Specific examples include itaconic acid, maleic acid, methacrylic acid, 2-chloroacrylic acid, 2-cyanoacrylic acid, aconitic acid, mesaconic acid, fumaric acid, glutaconic acid, citraconic acid, utraconic acid, and an anhydride of the unsaturated dicarboxylic acids. Itaconic acid and maleic acid are preferred.

Furthermore, a mixture comprising acrylic acid may further comprise copolymerizable monomers which do not have a carboxylic acid functionality or an anhydride thereof, whereby it is preferable that the proportion of the unsaturated carboxylic acid units is 50% by mol or more of the entire structural units. Preferably, the linear polyacidic acrylic polymer contains from 50 to 100 mole percent of acrylic acid repeating units.

The copolymerizable monomer is preferably an ethylenically unsaturated polymerizable monomer, and the copolymerizable monomer includes, for example, styrene, acrylamide, acrylonitrile, methyl methacrylate, vinyl chloride, allyl chloride, vinyl acetate, 1,1,6-trimethylhexamethylene dimethacrylate ester.

Among these linear polyacidic acrylic polymer of the formula (I), the homopolymers of acrylic acid and copolymers of acrylic acid and itaconic acid anhydride are preferred. According to a preferred embodiment, the linear polyacidic acrylic polymer is polyacrylic acid or a copolymer of acrylic acid and itaconic anhydride.

When the linear polyacidic acrylic polymer of the formula (I) has a weight-average molecular weight of less than 10 kDa , the strength of the cured dental resin-modified glass ionomer composition is lowered. On the other hand, when these polyalkenic acids have a weight-average molecular weight exceeding a viscosity of 300 kDa , upon mixing and blending the dental resin-modified glass ionomer composition becomes harder, so that workability is lowered in some cases. Therefore, the preferred weight-average molecular weight of the polyalkenic acid is from 10 to 300 kDa.

The process according to the present invention comprises a step (b) of reacting the linear polyacidic acrylic polymer with one or more polymerizable compounds of the following formula (II) in a solvent:

R-X (II)

wherein
X is selected from an amino group and an isocyanato group; and
R is an organic group having one or more polymerizable double bond;
for preparing a polymerizable linear polyacidic acrylic polymer having polymerizable pendant groups linked to the acrylic polymer backbone by amide groups.

The coupling according to step (b) serves to introduce one or more polymerizable moieties into the linear polyacidic acrylic polymer, which moieties can be post-polymerized to provide additional covalent crosslinking, imparting additional strength to the dental material comprising the polymer. According to the present invention, it is not required that the carboxylic acid groups of the polymer are protected. Therefore, the polymerizable linear polyacidic acrylic polymer having polymerizable pendant groups linked to the acrylic polymer backbone by amide groups can be used as a polymer according to the present invention without further treatment. In an alternative embodiment, the carboxylic acid groups of the polymer are protected. However, the carboxylic acid groups would have to be deprotected before the polymer may be used in a cement reaction. Therefore, the alternative embodiment is less preferred.

According to a preferred embodiment, R in formula (II) is a moiety of the following formula (III): 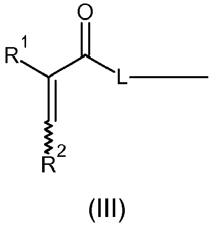 Wherein
R¹ represents a hydrogen atom, a carboxylic acid group or a C₁₋₃ alkyl group;
R² represents a hydrogen atom, a carboxylic acid group or a C₁₋₃ alkyl group;
L represents a divalent organic linker group.

In formula (III), L is preferably a group

-YL'-,

wherein
Y represents O or NH, and
L' represents a divalent organic group.

In formula (III), X is preferably an amino group and the carboxylic acid groups of the linear polyacidic acrylic polymer are activated with a coupling agent prior to the reaction with the polymerizable compounds of the formula (II). According to a preferred embodiment, the coupling agent is a carbodiimide. Specifically, the carbodiimide may be selected from N,N'-dicyclohexylcarbodiimide (DCC), N-(3-Dimethylaminopropyl)-N'-ethylcarbonate (EDC), and N,N'-diisopropylcarbodiimide (DIC).

In formula (III), when X is an isocyanato group, addition of a carboxylic acid to the isocyanate initially yields the mixed acid anhydride, decarboxylation of which leads to the N-substituted amide.

In the process according to the present invention, preferably 0.02 to 0.5 eq. of the one or more polymerizable compounds of the formula (II) are reacted based on the total number of carboxylic acid groups of the linear polyacidic acrylic polymer.

The reaction conditions of the reaction according to step b) of the present invention are not particularly limited. Accordingly, it is possible to carry out the reaction in any suitable solvent or a suitable mixture of two or more solvents. Preferably, a solvent may be selected from the group of dimethylformamide (DMF), acetonitrile, carbon tetrachloride, tetrahydrofurane (THF), and dioxane. More preferably, dimethylformamide (DMF), acetonitrile, and/or carbon tetrachloride are used.

The reaction temperature is not particularly limited. Preferably, the reaction is carried out at a temperature of between -10°C to the boiling point of the solvent. Preferably, the reaction temperature is in the range of from 0°C to 100°C. The reaction time is not particularly limited. Preferably, the reaction time is in the range of from 10 minutes to 120 hours, more preferably 1 hour to 80 hours. The reaction between the linear polyacidic acrylic polymer having repeating units of the formula (I) and the one or more polymerizable compound of the formula (II) may preferably be carried out at a temperature of from 20 to 100 °C for 1 to 60 hours.

The reaction product obtained in step b) may be isolated by precipitation and filtration. The product may be purified by washing with a suitable solvent.

The process of the present invention uses as a component of the dental resin-modified glass ionomer composition a specific polymerizable polyacidic polymer being a linear polyacidic acrylic polymer having polymerizable pendant groups linked to the acrylic polymer backbone by amide groups which preferably has repeating units of the following formula (IV) 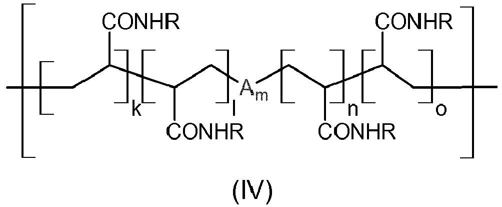 wherein
R is as defined in claim 1,
k, l, m, n, and o are independently integers of at least 0,
k+l+m +n+o is at least 2; and
at least one of k, l, n, and o is at least 1;
A which may be the same or different, independently represent a group selected from groups of the following formula (IVc), (IVd), and (IVf): 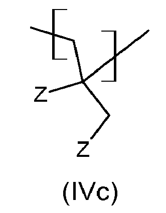 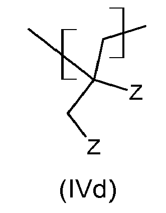 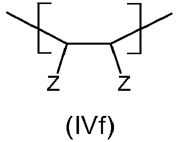 wherein Z is COOH or CONHR' (wherein R' is as defined in claim 1), and
   wherein the polyacidic polymer has a weight average molecular weight of 12 to 400 kDa.

The process according to the present invention comprises a step (c) of combining the polymerizable linear polyacidic acrylic polymer having polymerizable pendant groups linked to the acrylic polymer backbone by amide groups with a reactive dental glass capable of reacting with the polyacidic acrylic polymer in a cement reaction. "Combining" according to step (c) includes physical mixing of the components and the association of the components such that the physical mixing of the components is facilitated, preferably in a single step. Accordingly, the polymerizable linear polyacidic acrylic polymer and the reactive dental glass may be combined in a kit-of-parts or two- or multi-pack composition wherein the polymerizable linear polyacidic acrylic polymer and the reactive dental glass are separated for storage.

The formation of a dental resin-modified glass ionomer composition, which is useful as a dental material, is based on a reaction in the presence of water between the polyacidic acrylic polymer and the reactive dental glass in a cement reaction. Water provides a medium needed for the ionic acid-base reaction to take place between the polyacidic acrylic polymer and the reactive dental glass. According to embodiments of the present invention, water may be present in an amount from about 0.5 wt% to about 40 wt% based on the total weight of the dental resin-modified glass ionomer composition. For example, in one embodiment, water may be present in an amount from 1.0 wt% to 30 wt% of the dental resin-modified glass ionomer composition. In another embodiment, water may be present in an amount from 2.0 wt% to 25 wt% of the dental resin-modified glass ionomer composition.

A dental resin-modified glass ionomer composition according to the present invention, comprises the polyacidic acrylic polymer, a reactive dental glass capable of reacting with the polyacidic acrylic polymer in a cement reaction, and optionally a an initiator system, particulate non-reactive filler, one or more additional monomers or crosslinkers and further additives.

Examples of a reactive dental glass include materials commonly known in the art of dental compositions such as calcium or strontium-containing and aluminum-containing materials. Preferably, particulate reactive fillers contain leachable fluoride ions. Specific examples of a reactive dental glasses are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass. Suitable a reactive dental glasses further include metal oxides such as zinc oxide and magnesium oxide, and ion-leachable glasses, e.g., as described in US-A 3,655,605, US-A 3,814,717, US-A 4,143,018, US-A 4,209,434, US-A 4,360,605 and US-A 4,376,835.

Preferably, the reactive particulate glass is a reactive particulate glass comprising:
1) 20 to 45% by weight of silica,
2) 20 to 40% by weight of alumina,
3) 20 to 40% by weight of strontium oxide,
4) 1 to 10% by weight of P₂O₅, and
5) 3 to 25% by weight of fluoride.

The reactive particulate glass usually has an average particle size of from 0.005 to 100 µm, preferably of from 0.01 to 40 µm as measured, for example, by electron microscopy or by using a conventional laser diffraction particle sizing method as embodied by a MALVERN Mastersizer S or MALVERN Mastersizer 2000 apparatus. The reactive particulate glass may be a multimodal the reactive particulate glass representing a mixture of two or more particulate fractions having different average particle sizes. The reactive particulate glass may also be a mixture of particles of different chemical composition. The reactive particulate glass may be surface modified by a surface modifying agent.

The present dental resin-modified glass ionomer composition preferably comprises 20 to 90 percent by weight of the reactive particulate glass, more preferably 30 to 80 percent by weight, based on the total weight of the composition.

Suitable non-reactive fillers may be selected from fillers currently used in dental restorative compositions. The filler may have a unimodal or polymodal (e.g., bimodal) particle size distribution. The filler can be an inorganic material. It can also be a crosslinked organic material that is insoluble in the polymerizable resin, and is optionally filled with inorganic filler. The filler can be radiopaque, radiolucent or non-radiopaque. Examples of suitable non-reactive inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents includes gamma- methacryloxypropyltrimethoxysilane, gamma-mercaptopropyltriethoxysilane, gamma- aminopropyltrimethoxysilane, and the like.

The process according to the present invention may further comprise combining an initiator system and optionally one or more polymerizable monomers or crosslinkers to the dental resin-modified glass ionomer composition.

Accordingly, the dental resin-modified glass ionomer composition preferably comprises a polymerization initiator system. As a polymerization initiator system, any compound or system capable of initiating the polymerization reaction according to the present invention may be used. The polymerization initiator may be a photoinitiator or a redox initiator or a mixture thereof.

A suitable redox initiator comprises one or more reducing agents and one or more oxidizing agents, which typically react with or otherwise cooperate with one another to produce free-radicals capable of initiating polymerization of polymerizable double bonds in a dark reaction, independent from the presence of light. The reducing and oxidizing agents are selected so that the polymerization initiator system is sufficiently storage-stable and free of undesirable colorization to permit storage and use under typical dental conditions. Moreover, the reducing and oxidizing agents are selected so that the polymerization initiator system is sufficiently miscible with the resin system to permit dissolution of the polymerization initiator system in the composition.

Useful reducing agents include ascorbic acid, ascorbic acid derivatives, and metal complexed ascorbic acid compounds as described in US 5,501,727; amines, namely tertiary amines, such as 4-tert-butyl dimethylaniline; aromatic sulfinic salts, such as p-toluenesulfinic salts and benzenesulfinic salts; thioureas, such as 1-ethyl-2-thiourea, tetraethyl thiourea, tetramethyl thiourea, 1,1-dibutyl thiourea, and 1,3-dibutyl thiourea; and mixtures thereof. Other secondary reducing agents may include cobalt (II) chloride, ferrous chloride, ferrous sulfate, hydrazine, hydroxylamine, salts of a dithionite or sulfite anion, and mixtures thereof.

Suitable oxidizing agents include persulfuric acid and salts thereof, such as ammonium, sodium, potassium, cesium, and alkyl ammonium salts. Additional oxidizing agents include peroxides such as benzoyl peroxides, hydroperoxides such as cumyl hydroperoxide, t-butyl hydroperoxide, and amyl hydroperoxide, as well as salts of transition metals such as cobalt (III) chloride and ferric chloride, cerium (IV) sulfate, perboric acid and salts thereof, permanganic acid and salts thereof, perphosphoric acid and salts thereof, and mixtures thereof. One or more different oxidizing agents or one or more different reducing agent may be used in the polymerization initiator system. Small quantities of transition metal compounds may also be added to accelerate the rate of redox cure. The reducing and oxidizing agents are present in amounts sufficient to permit an adequate free-radical reaction rate.

A suitable redox initiator may be selected from benzoyl peroxide/ amine, potassium peroxodisulfate (K₂S₂O₈)/ascorbinic acid, sodium peroxodisulfate, sodium pyrosulfite (Na₂S₂O₅).

The reducing or oxidizing agents may be microencapsulated for enhancing shelf stability of the composition, and if necessary permitting packaging the reducing and oxidizing agents together (US 5,154,762). Appropriate selection of an encapsulant may allow combination of the oxidizing and reducing agents and even of an acid-functional component and optional filler in a storage-stable state. Moreover, appropriate selection of a water-insoluble encapsulant allows combination of the reducing and oxidizing agents with the particulate reactive glass and water in a storage-stable state.

Suitable photoinitiators for polymerizing free radically photopolymerizable compositions may include binary and tertiary systems. Tertiary photoinitiators may include an iodonium salt, a photosensitizer, and an electron donor compound as described in US 5,545,676. Suitable iodonium salts include the diaryl iodonium salts, e.g., diphenyliodonium chloride, diphenyliodonium hexafluorophosphate, diphenyliodonium tetrafluoroborate, and tolylcumyliodonium tetrakis(pentafluorophenyl)borate. Suitable photosensitizers are monoketones and diketones that absorb some light within a range of about 400 nm to about 520 nm (preferably, about 450 nm to about 500 nm). Particularly suitable compounds include alpha diketones that have some light absorption within a range of about 400 nm to about 520 nm (even more preferably, about 450 to about 500 nm). Examples include camphorquinone, benzil, furil, 3,3,6,6-tetramethylcyclo-hexanedione, phenanthraquinone, 1-phenyl-1,2-propanedione and other 1-aryl-2-alkyl-1,2-ethanediones, and cyclic alpha diketones. Suitable electron donor compounds include substituted amines, e.g., ethyl dimethylaminobenzoate.

Suitable photoinitiators may also include phosphine oxides typically having a functional wavelength range of about 380 nm to about 1200 nm. Examples of phosphine oxide free radical initiators with a functional wavelength range of about 380 nm to about 450 nm include acyl and bisacyl phosphine oxides such as those described in US 4,298,738, US 4,324,744 US and 4,385,109 and EP 0 173 567. Specific examples of the acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphonate, and 2,4,6-trimethylbenzoylethoxyphenylphosphine oxide. Commercially available phosphine oxide photoinitiators capable of free-radical initiation when irradiated at wavelength ranges of greater than about 380 nm to about 450 nm include bis(2,4,6-trimethylbenzoyl)phenyl phosphine oxide (IRGACURE 819), bis(2,6-dimethoxybenzoyl)-(2,4,4-trimethylpentyl) phosphine oxide (CGI 403), a 25:75 mixture, by weight, of bis(2,6-dimethoxybenzoyl)-2,4,4-trimethylpentyl phosphine oxide and 2-hydroxy-2-methyl-1-phenylpropan-1-one (IRGACURE 1700), a 1:1 mixture, by weight, of bis(2,4,6-trimethylbenzoyl)phenyl phosphine oxide and 2-hydroxy-2-methyl-1-phenylpropane-1-one (DAROCUR 4265), and ethyl 2,4,6-trimethylbenzylphenyl phosphinate (LUCIRIN LR8893X). Typically, the phosphine oxide initiator is present in the composition in catalytically effective amounts, such as from 0.1 percent by weight to 5.0 percent by weight, based on the total weight of the composition.

Tertiary amine reducing agents may be used in combination with an acylphosphine oxide Examples of suitable aromatic tertiary amine include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, 4-N,N-dimethylaminobenzoic acid ethyl ester, 4-N,N-dimethylaminobenzoic acid methyl ester, 4-N,N-dimethylaminobenzoic acid n-butoxyethyl ester, 4-N,N-dimethylaminobenzoic acid 2-(methacryloyloxy) ethyl ester, 4-N,N-dimethylaminobenzophenone ethyl 4-(N,N-dimethylamino)benzoate and N,N-dimethylaminoethyl methacrylate. Examples of an aliphatic tertiary amine include trimethylamine, triethylamine, N-methyldiethanolamine, N-ethyldiethanolamine, N-n-butyldiethanolamine, N-lauryldiethanolamine, triethanolamine, 2-(dimethylamino) ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

The amine reducing agent may be present in the composition in an amount from 0.1 percent by weight to 5.0 percent by weight, based on the total weight of the composition.

The amount of active species of the polymerization initiator is not particularly limited. Suitably, the amount of polymerization initiator in the polymerization system is in the range of from 0.001 to 5 mol % based on the total amount of the monomers.

A suitable monomer or crosslinker contains at least one polymerizable functional group. Preferably, a monomer having a single polymerizable double bond is hydrolysis-stable and water-soluble. The term "hydrolysis-stable" means that the monomer is stable to hydrolysis in an acidic medium, such as in a dental composition. A hydrolysis-stable, water-soluble monomer may polymerize together with the linear polyacidic acrylic polymer having polymerizable pendant groups linked to the acrylic polymer backbone by amide groups in the presence of a polymerization initiator system. Accordingly, the monomer may polymerize with itself and/or with the polymerizable pendant groups of the linear polyacidic acrylic polymer. Hence, besides of the formation of a polymer formed of the monomer, there is a graft polymerization wherein monomer(s) react with the polymerizable pendant groups of the linear polyacidic acrylic polymer, whereby a graft polymer is formed. Furthermore, the graft side chains formed of the monomer may additionally react with the pendant polymerizable groups of another linear polyacidic acrylic polymer according, whereby a crosslinked polymer may be obtained.

In a preferred embodiment of the aqueous dental glass ionomer composition, the hydrolysis-stable, water-soluble monomer having a single polymerizable double bond has a carboxylic acid group and is a compound represented by the general formula (V):

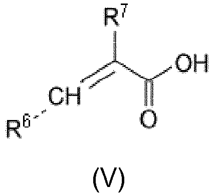

In formula (V), R⁶ is a hydrogen atom or a straight chain or branched C₁₋₃ alkyl group, and R⁷ is a hydrogen atom or a straight-chain or branched C₁₋₆ alkyl group which may be substituted by a -COOH group. In formula (V), the dotted line indicates that R⁶ may be in either the cis or trans orientation. Preferably, R⁶ is a hydrogen atom, and R⁷ is a hydrogen atom or a 6₁₋₃ alkyl group optionally substituted with a -COOH group. More preferably, R⁶ is a hydrogen atom, and R⁷ is a hydrogen atom or a methyl group substituted with a -COOH group, that is compound of formula (V) is acrylic acid or itaconic acid. Most preferably, the compound of formula (V) is acrylic acid.

In formula (V), residues R⁶ and R⁷ may be selected with the proviso that the molecular weight of the monomer having one polymerizable double bond is at most 200 Da, preferably at most 150 Da, more preferably at most 100 Da.

Furthermore, the hydrolysis-stable, water-soluble monomer having a single polymerizable double bond may be 2-hydroxyethyl methacrylate (HEMA), hydroxypropyl methacrylate, 2-hydroxyethyl acrylamide (HEAA), N,N-dimethyl(meth)acrylamide, N,N-diethyl(meth)acrylamide, N,N-di-n-propyl(meth)acrylamide, and N-ethyl-N-methyl(meth)acrylamide. Further monomers include alpha, beta-unsaturated monomers such as methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, hydroxypropyl acrylate, hydroxypropyl methacrylate, tetrahydrofurfuryl acrylate, tetrahydrofurfuryl methacrylate, glycidyl acrylate, glycidyl methacrylate, glycerol monoacrylate, and glycerol monomethacrylate.

The monomer is preferably selected in view of a good processability and applicability of the final aqueous dental glass ionomer composition, in particular in terms of viscosity. Therefore, the viscosity of the monomer is preferably in the range of 0.1 to 100 mPa·s, more preferably 0.3 to 50 mPa·s, even more preferably 0.5 to 25 mPa·s, yet even more preferably 0.8 to 10 mPa·s, in particular 0.9 to 3 mPa·s.

Monomers comprising a carboxylic acid group are particularly advantageous, since such monomers introduce additional carboxylic acid groups into the acidic polymer in the aqueous dental glass ionomer composition, which can take part in the cement reaction resulting in a further improved setting or curing reaction in the presence of a reactive particulate glass.

Preferably, the monomer is contained in the dental resin-modified glass ionomer composition in an amount of from 0.1 to 20, more preferably 1 to 15 even more preferably 2 to 10 percent by weight based on the total weight of the aqueous dental glass ionomer composition.

The dental resin-modified glass ionomer composition according to the present invention may further contain a crosslinker, which is preferably a polymerizable hydrolysis-stable crosslinker having at least two polymerizable double bonds.

The crosslinker may be an alkylenediol dimethylacrylate such as 1,3-butanediol dimethacrylate, 1,4-butanediol dimethacrylate, an alkylenediol divinyl ether such as 1,4-butanediol divinyl ether, di(ethylene glycol) dimethacrylate, di(ethylene glycol) divinyl ether, pentaerythritol diacrylate monostearate, ethylene glycol dimethacrylate, trimetylolpropane trimethacrylate, pentaerythritol triacrylate or triallyl ether, pentaerythritol tetraacrylate and trimetylolpropane triacrylate. The crosslinker may also be 1,3-Bis(acrylamido)-N,N'-diethylpropane, N,N-Di(cyclopropyl acrylamido) propane.

Preferably, the crosslinker is a polymerizable compound of the following formula (VI), which is disclosed in EP2705827 and WO2014040729:

A"-L(B)_{n'} (VI)

wherein
A" is a group of the following formula (VII) 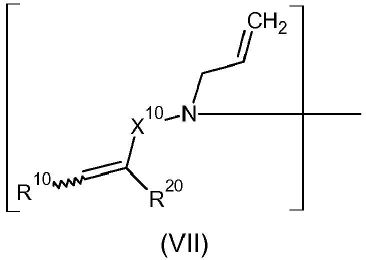
   X¹⁰ is CO, CS, CH₂, or a group [X¹⁰⁰Z¹⁰]ₖ, wherein X¹⁰⁰ is an oxygen atom, a sulfur atom or NH, Z¹⁰ is a straight chain or branched C₁₋₄ alkylene group, and k is an integer of from 1 to 10;
   R¹⁰ is a hydrogen atom,
      -COOM¹⁰,
      a straight chain or branched C₁₋₁₆ alkyl group which may be substituted by a C₃₋₆ cycloalkyl group, a C₆₋₁₄ aryl or C₃₋₁₄ heteroaryl group, -COOM¹⁰, -PO₃M¹⁰, -O-PO₃M¹⁰₂ or -SO₃M¹⁰,
      a C₃₋₆ cycloalkyl group which may be substituted by a C₁₋₁₆ alkyl group, a C₆₋₁₄ aryl or C₃₋₁₄ heteroaryl group, -COOM¹⁰, -PO₃M¹⁰, -O-PO₃M¹⁰₂ or -SO₃M¹⁰,
      a C₆₋₁₄ aryl or C₃₋₁₄ heteroaryl group which may be substituted by -COOM¹⁰, - PO₃M¹⁰, -O-PO₃M¹⁰₂ or -SO₃M¹⁰,
   R²⁰ is a hydrogen atom,
      -COOM¹⁰
      a straight chain or branched C₁₋₁₆ alkyl group which may be substituted by a C₆₋₁₄ aryl or C₃₋₁₄ heteroaryl group, -COOM¹⁰, -PO₃M¹⁰, -O-PO₃M¹⁰₂ and - SO₃M¹⁰,
      a C₃₋₆ cycloalkyl group which may be substituted by a C₁₋₁₆ alkyl group, a C₆₋₁₄ aryl or C₃₋₁₄ heteroaryl group, -COOM¹⁰, -PO₃M¹⁰, -O-PO₃M¹⁰₂ or -SO₃M¹⁰, or
      a C₆₋₁₄ aryl or C₃₋₁₄ heteroaryl group which may be substituted by -COOM¹⁰, - PO₃M¹⁰, -O-PO₃M¹⁰₂ and -SO₃M¹⁰,
L is a single bond or a linker group;
B independently is
   a group according to the definition of A",
   a group of the following formula (VIII) 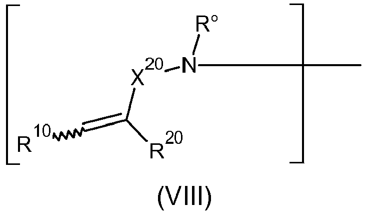 wherein
   X²⁰ independently has the same meaning as defined for X¹ in formula (VII),
   R¹⁰ and R²⁰ are independent from each other and independently have the same meaning as defined for formula (VII),
   R° is a hydrogen atom,
      a straight chain or branched C₁₋₁₆ alkyl group which may be substituted by a C₃₋₆ cycloalkyl group, a C₆₋₁₄ aryl or C₃₋₁₄ heteroaryl group, - COOM¹⁰, -PO₃M¹⁰, -O-PO₃M¹⁰₂ or -SO₃M¹⁰,
      a C₃₋₆ cycloalkyl group which may be substituted by a C₁₋₁₆ alkyl group,
      a C₆₋₁₄ aryl or C₃₋₁₄ heteroaryl group, -COOM¹⁰, -PO₃M¹⁰, -O-PO₃M¹⁰₂ or -SO₃M¹⁰,
      a C₆₋₁₄ aryl group which may be substituted by -COOM¹⁰, -PO₃M¹⁰, -O-PO₃M¹⁰₂ or -SO₃M¹⁰,
   a group of the following formula (IX) 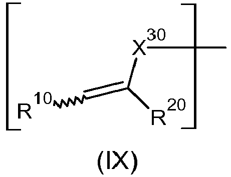 wherein
   X³⁰ is CO, -CH₂CO-, CS, or -CH₂CS-,
   R¹⁰ and R²⁰ which are independent from each other and independently have the same meaning as defined for formula (VII), or
   a group

   [X⁴⁰Z²⁰⁰]ₚE,

   wherein
   Z²⁰⁰ is a straight chain or branched C₁₋₄ alkylene group,
   X⁴⁰ is an oxygen atom, a sulfur atom or NH,
   E is a hydrogen atom,
      PO₃M₂,
      a straight chain or branched C₁₋₁₆ alkyl group which may be substituted by a C₃₋₆ cycloalkyl group, a C₆₋₁₄ aryl or C₃₋₁₄ heteroaryl group, -COOM¹⁰, -PO₃M¹⁰, -O-PO₃M¹⁰₂ or - SO₃M¹⁰,
      a C₃₋₆ cycloalkyl group which may be substituted by a C₁₋₁₆ alkyl group, a C₆₋₁₄ aryl or C₃₋₁₄ heteroaryl group, - COOM¹⁰, -PO₃M¹⁰, -O-PO₃M¹⁰₂ or -SO₃M¹⁰,
      a C₆₋₁₄ aryl or C₃₋₁₄ heteroaryl group which may be substituted by -COOM¹⁰, -PO₃M¹⁰, -O-PO₃M¹⁰₂ or - SO₃M¹⁰, and
   p is an integer of from 1 to 10;
   and
n' is an integer of from 1 to 4;
wherein M¹⁰ which are independent from each other each represent a hydrogen atom or a metal atom. Preferably, when L is a single bond, B cannot be a group according to the definition of A" or a group of the formula (VIII).

The following groups are preferred groups of formula (VII), wherein M is a hydrogen atom or a metal atom:

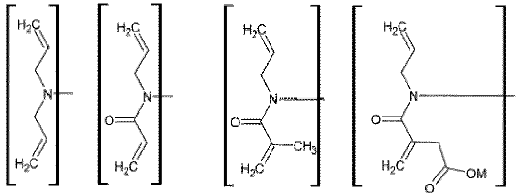

Preferred divalent linker groups may be selected from methylene, ethylene, propylene, butylene and the following divalent groups:

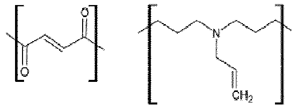

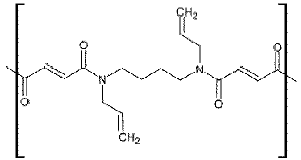

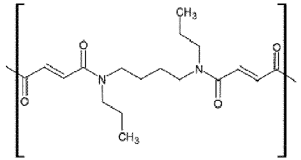

N,N'-(2E)-but-2-en-1,4-diallylbis-[(N-prop-2-en-1) amide and N,N-di(allyl acrylamido) propane are preferred.

Further crosslinkers include alpha, beta-unsaturated crosslinkers such as the diglycidyl methacrylate of bis-phenol A ("bis-GMA"), glycerol diacrylate, glycerol dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, di-, tri-, and tetraacrylates and methacrylates of pentaerythritol and dipentaerythritol, 1,3-butanediol diacrylate, 1 ,3-butanediol dimethacrylate, 1 ,4-butanedioldiacrylate, 1 ,4-butanediol dimethacrylate, 1 ,6-hexane diol diacrylate, 1 ,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4- cyclohexyl carbamate, di-1 -methyl-2-methacryloxyethyl-trimethyl-hexamethylene dicarbamate, di-1-methyl-2-methacryloxyethyl-dimethylbenzene dicarbamate, di-1-methyl-2- methacryloxyethyl-dimethylcyclohexane dicarbamate, methylene-bis-1 -methyl-2- methacryloxyethyl-4-cyclohexyl carbamate, di-1 -chloromethyl-2-methacryloxyethyl- hexamethylene dicarbamate, di-1 -chloromethyl-2-methacryloxyethyl- trimethylhexamethylene dicarbamate, di-1 -chloromethyl-2-methacryloxyethyl- dimethylbenzene dicarbamate, di-1 -chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1 -methyl-2-methacryloxyethyl-trimethylhexamethylene dicarbamate, di-1 -methyl-2-methacryloxyethyl-dimethylbenzene dicarbamate, di-1 -methyl-2-metha-cryloxyethyl-dimethylcyclohexane dicarbamate, methylene-bis-1 -methyl-2-methacryloxyethyl-4-cyclohexyl carbamate, di-1-chloromethyl-2- methacryloxyethyl-hexa methylene dicarbamate, di-1 -chloromethyl-2-methacryloxyethyl- trimethylhexamethylene dicarbamate, di-1-chloromethyl-2-methacryloxyethyl- dimethylbenzene dicarbamate, di-1-chloromethyl-2-methacryloxyethyl-dimethylcyclohexane dicarbamate, methylene-bis-1 -chloromethyl-2-methacryloxyethyl-4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxyphenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2,1-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)- 2-hydroxypropane-1-acryalte]propane, may be mentioned. Other suitable examples of polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates. Mixtures of alpha, beta-unsaturated monomers can be added if desired.

Preferably, the mixed but unset dental resin-modified glass ionomer composition of the invention will contain a combined weight of about 0.5 to about 50%, more preferably about 1 to about 40%, and most preferably about 5 to 35% water, solvents, diluents and alpha, beta-unsaturated monomers and crosslinkers, based on the total weight (including such water, solvents, diluents and alpha, beta-unsaturated monomers and crosslinkers) of the mixed but unset dental composition components.

A dental resin-modified glass ionomer composition according to the present invention may also include a modifying agent such as tartaric acid, for adjusting the working time and a setting time of the glass ionomer cement reaction, respectively, when preparing the cement as described in US-A 4,089, 830, US-A 4, 209,434, US-A 4,317, 681 and US-A 4,374, 936. In general, an increase in working time results in an increase in setting time as well.

The "working time" is the time between the beginning of the setting reaction when the polymer and modified particulate reactive filler are combined in the presence of water, and the time the setting reaction proceeds to the point when it is no longer practical to perform further physical work upon the system, e.g. spatulate it or reshape it, for its intended dental or medical application.

The "setting time" is the time measured from the beginning of the setting reaction in a restoration to the time sufficient hardening has occurred to allow subsequent clinical or surgical procedures to be performed on the surface of the restoration.

In a setting reaction, due to the presence of polymerizable double bonds, a polymerization reaction takes place.

The present invention also provides a dental resin-modified glass ionomer composition obtainable according to the process of the present invention. The dental resin-modified glass ionomer composition of the present invention can be prepared by combining all the components using conventional mixing techniques. The dental resin-modified glass ionomer composition may be partially or fully hardened by an ionic reaction between the polymerizable linear polyacidic acrylic polymer having polymerizable pendant groups linked to the acrylic polymer backbone by amide groups and the reactive dental glass capable of reacting with the polyacidic acrylic polymer in a cement reaction. Additionally, the dental resin-modified glass ionomer composition of the present invention may contain a polymerizable component and a photoinitiator and be hardened by photoinitiation, or may be partially or fully hardened by chemical polymerization such as a redox cure system in which the composition contains a free-radical initiator system, e.g., including an oxidizing agent and a reducing agent. Alternatively, the dental resin-modified glass ionomer composition of the present invention may contain different initiator systems, such that the composition can be both a photopolymerizable and a chemically polymerizable composition, as well as an ionically hardenable composition.

The dental resin-modified glass ionomer composition of the present invention can be provided as a one-pack system or multi-pack system, e.g., two-pack powder/liquid, paste/liquid, paste/powder and paste/paste systems. Other forms employing multi-pack combinations, each of which is in the form of a paste, powder, liquid, or gel, are also possible. The dental resin-modified glass ionomer composition is preferably a two-pack composition.

The components of the dental resin-modified glass ionomer composition of the present invention may be divided up into separate packs. However, the polymerizable linear polyacidic acrylic polymer and the reactive dental glass capable of reacting with the polyacidic acrylic polymer in a cement reaction would not all be present in the same pack, although any two of these may be grouped together in the same part along with any combination of other components. Furthermore, in a redox multi-pack system, one pack typically contains the oxidizing agent and another pack typically contains the reducing agent. However, the reducing agent and oxidizing agent could be combined in the same pack of the system if the components are kept separated, e.g. through use of microencapsulation.

In one embodiment, the dental resin-modified glass ionomer composition of the present invention is provided as a two-pack, paste-paste system. The first pack, Paste A, may contain the reactive dental glass, water, reducing agent, and photoinitiator. Further optional components may be added to Paste A. The second pack, Paste B, may contain the polymerizable linear polyacidic acrylic polymer having polymerizable pendant groups linked to the acrylic polymer backbone by amide groups and an oxidizing agent. Paste B may further contain a monomer or crosslinker component, nonreactive fillers, a light cure catalyst and further optional components. The combination of ingredients in Paste A and Paste B generally provides a stable dental resin-modified glass ionomer composition with adhesion to dentin and enamel, radiopacity for x-ray diagnosis, and good aesthetics. Such compositions are especially useful for bulk filling of tooth restorations by a convenient, one-step, easy mix direct restoration method.

In some embodiments, two-pack dental resin-modified glass ionomer composition can be provided in a dual barrel syringe having a first barrel and a second barrel, wherein the Paste A resides in the first barrel and the Paste B resides in the second barrel. In other embodiments, two-pack dental compositions of the present invention can be provided in a unit-dose capsule. In some embodiments, each part of a multipart dental system can be mixed together using a static mixer.

The components of the dental resin-modified glass ionomer composition can be included in a kit, where the contents of the composition are packaged to allow for storage of the components until they are needed.

When used as a dental resin-modified glass ionomer composition, the components of the dental resin-modified glass ionomer composition can be mixed and clinically applied using conventional techniques. A curing light is required for the initiation of photopolymerizable compositions.

The present dental resin-modified glass ionomer composition is a curable dental composition. A cured dental resin-modified glass ionomer composition can be obtained therefrom. When cured, the present dental resin-modified glass ionomer composition preferably has the following properties:
- the cured composition's adhesive bond strength to dentin is of at least 5 MPa as measured according to ISO 29022:2013; and
- the cured composition's flexural strength is of at least 80 MPa as measured according to ISO 4049.

### Particularly preferred embodiments of the dental resin-modified glass ionomer composition

According to a particularly preferred embodiment, the dental resin-modified glass ionomer composition according to the invention comprises
(A) a reactive particulate glass comprising
   1) 20 to 45% by weight of silica,
   2) 20 to 40% by weight of alumina,
   3) 20 to 40% by weight of strontium oxide,
   4) 1 to 10% by weight of P₂O₅, and
   5) 3 to 25% by weight of fluoride,
(B) a polymerizable polyacidic polymer obtainable by a process comprising the following steps:
   (a) providing a linear polyacidic acrylic polymer having repeating units of the following formula (I) 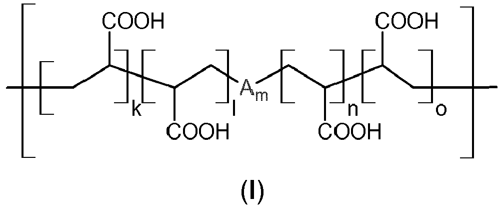 wherein
      A , which may be the same or different, independently is selected from a group of the following formula (Ia) to (If): 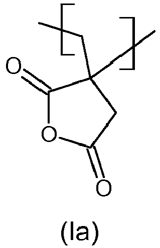 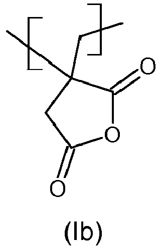 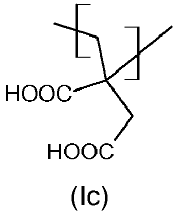 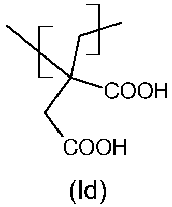 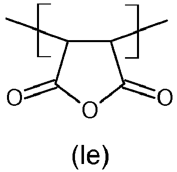 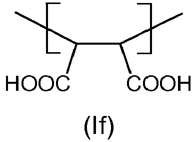
      k, l, m, n and o are independently integers of at least 0,
      k+l+m+n+o is at least 2; and
      at least one of k, l, n, and o is at least 1;
      wherein the polyacidic polymer has a weight average molecular weight of 10 to 300 kDa;
   (b) reacting the linear polyacidic acrylic acid polymer with one or more polymerizable compounds of the following formula (II) in a solvent:

      R-X (2)

      wherein
      X is selected from an amino group and an isocyanato group; and
      R is an organic group having one or more polymerizable double bond;
      for preparing a polymerizable linear polyacidic acrylic polymer having polymerizable pendant groups linked to the acrylic polymer backbone by amide groups;
(C) a hydrolysis-stable, water-soluble monomer having one polymerizable double bond and a carboxylic acid group, said monomer having a molecular weight of at most 200 Da is a compound represented by the general formula (4'): 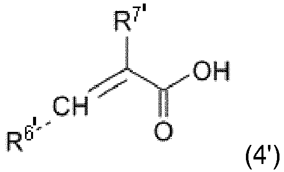 wherein
   R^{6'} is a hydrogen atom or a straight chain or branched C₁₋₃ alkyl group, and
   R^{7'} is a hydrogen atom or a straight-chain or branched C₁₋₃ alkyl group which may be substituted by a - COOH group, wherein R^{6'} and R^{7'} are selected with the proviso that the molecular weight of the compound of formula (4) is at most 200 Da;
   preferably,
   R^{6'} is a hydrogen atom, and
   R^{7'} is a hydrogen atom or a C₁₋₃ group optionally substituted with a -COOH group;
   more preferably,
   R^{6'} is a hydrogen atom, and
   R^{7'} is hydrogen atom or a methyl group substituted with a -COOH group;
(D) a polymerization initiator system being based on a radical initiator in the form of a photoinitiator or a redox initiator or a mixture thereof, and
(E) a polymerizable hydrolysis-stable crosslinker having at least two polymerizable carbon-carbon double bonds.

The aqueous dental glass ionomer composition according to the present invention may, besides of optional component, comprise additional optional components.

The dental resin-modified glass ionomer composition according to the present invention may contain a non-reactive filler and/or further components such as an inhibitor or a sensitizer, solvents, pigments, free radical scavengers, polymerization inhibitors, nonreactive diluents , surfactants (such as to enhance solubility of an inhibitor e. g., polyoxyethylene), coupling agents to enhance reactivity of fillers e.g.,3-(trimethoxysilyl) propyl methacrylate, and rheology modifiers.

Suitable solvents or nonreactive diluents include alcohols such as ethanol and propanol. Suitable reactive diluents are alpha, beta unsaturated monomers for providing altered properties such as toughness, adhesion, and set time.

An example of a suitable free radical scavenger is 4-methoxyphenol. An example of a suitable inhibitor is tert.-butyl hydroquinone (TBHQ), hydroxytoluene or butylated hydroxytoluene (BHT). The amount of inhibitor may be selected from 0.001 to 2% and preferably from 0.02 to 0.5% based on the total weight of the copolymer/comonomer/water mixture.

The dental resin-modified glass ionomer composition according to the present invention may also include further components to improve the radio-opacity, such as CaWO₄, ZrO₂, YF₃ or to increase the fluoride release such as YF₃.

Preferably, the mixed but unset dental compositions of the invention will contain a combined weight of about 0.5 to about 50%, more preferably about 1 to about 40%, and most preferably about 5 to 35% water, solvents, diluents and alpha, beta-unsaturated monomers and crosslinkers, based on the total weight (including such water, solvents, diluents and alpha, beta-unsaturated monomers) of the mixed but unset aqueous dental glass ionomer composition components.

A mixture comprising the polymerizable polymer may be used for the preparation of a dental composition, preferably for the preparation of a cured dental composition, more preferably for the preparation of a cured aqueous dental glass ionomer composition.

The dental composition may be a dental material to be used in the oral cavity. Specifically, dental compositions according to the invention are useful in a variety of dental and orthodontic applications, including restorative and filling materials, luting cements, adhesive cements, base or orthodontic cements, cavity liners and bases, pit and fissure sealants, orthodontic adhesives, and dental coatings. The dental compositions may also be used to prepare a dental article by hardening to form, for example, dental mill blanks, dental crowns, dental fillings, dental prostheses, and orthodontic devices, in particular by any suitable process including 3D-printing.

The invention will now be further illustrated by the following Examples.

### Examples

### Example 1 - Polymer analogous modification of PAA

To a solution of 10 g (139 mmol) polyacrylic aicd (136 kDa) in 80 mL DMF at 60°C were added 3.1- 76.3 mmol (0.02 - 0.55 eq.) N,N'-dicyclohexyl carbodiimide (DCC) and stirred for 2 min. Then 2.8 - 69.4 mmol (0.02 - 0.50 eq.) N-(2-aminoethyl)-methacrylamido hydrochloride were added in a manner that the local precipitate does not agglomerates. Thereafter, the reaction mixture was stirred for 24 h at 60 °C and cooled down. The precipitating dicyclohexyl urea crystals were filtered off. The polymer solution was concentrated by distilling part of the solvent off and precipitated in 400 mL acetonitrile. The precipitate was filtered off, dissolved in 50 mL distilled water and dialyzed 4 days against 4 L distilled water (MWCO=1000 or 2500 g/mol, two-fold water exchange per day). Subsequently, the solution was filtered and the polymer was obtained by freeze drying of the polymer solution.

### Example 2 - Polymer analogous modification of PAA

10.89 g (0.120 mol) polyacrylic acid (136 kDa) and 1.38 g DCC (6.7 mmol, 0.06 equiv.) N,N'-dicyclohexyl carbodiimide (DCC) were dissolved in 100 mL DMF and stirred for 2-3 min. at 60°C. In parallel to 1.12 g (6.7 mmol, 0.06 equiv.) N-(2-aminoethyl)-methacrylamido hydrochloride dissolved in 10 ml DMF were added 0.930 ml (0.06 equiv.) triethylamine. This solution was added dropwise during 10 min. to the polyacid solution. Then the mixture was stirred for 20 h at 60 °C under light protection. Subsequently, the solvent was removed and the high viscous residue was added to 200 mL acetonitrile within 30 minutes. Thereafter, the turbid acetonitrile phase was decanted from solid parts. The residue was dissolved in 200 mL distilled water and freeze dried. The obtained modified polymer (12.85 g) shows an acrylate modification of 5-6 mol-% in relation to free carboxylic acid groups by ¹H-NMR spectra.

### Example 3 - Poly(acrylic acid-co-itaconic acid), modified with 2-[(2-methacryloyl) amino] ethylamine

10 g *Poly*(tert.-butylacrylate-*co*-itaconic acid anhydride) were dissolved in a 100 mL flask in 40 g Dioxane and 1 eq. Triethylamine in relation to the anhydride groups in the polymer. To this polymer solution 1.1 equivalents 2-[(2-Methacryloyl)amino] ethyl ammonium chloride in 10 ml Ethanol were added during 5 min and stirred for 2h. Then the ethanol was distilled off and the residue was dissolved in cold trifluoro acetic acid and stirred overnight. The formed colorless solid was separated and dissolved in acetonitrile. The solution was dropped into water. This reprecipitation was repeated twice. Then the solid polymer was dried in vacuum.

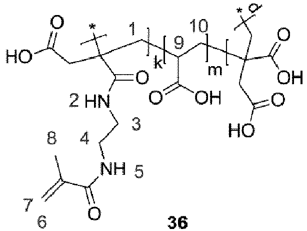

**Yield:** 62 - 72 %
**FT-IR (Diamante):** ṽ_{max, br} [cm⁻¹]: 2947, 1705 (vᵥₐₗ. C=O, carboxylic acid), 1654 (vᵥₐₗ. - C=O, Amide I), 1610 (vᵥₐₗ. -C=C), 1545 (vᵥₐₗ. -C=O, Amide II), 1451, 1239, 1170, 801, 604.
**¹H**-**NMR** (300 MHz, DMSO-d₆): δ (ppm): 12.4 (br, COOH), 8.0 (br, 2,5), 5.9 (br, 6), 5.3 (br, 7), 3.4 (br, 3,4), 2.2 (br, 9), 1.9 (br, 8), 1.7-1.1 (br, 1,10).
**DSC:** Tg= 117 °C

### Comparative Example 1 - Poly(acrylic acid-co-itaconic acid), modified with 2-hydroxy ethyl methacrylate

To a solution of 0.5 g (5.5 mmol) *poly*(acrylic acid-*co*-itaconic acid anhydride) in 5 mL dioxane were added 600 mg (4.6 mmol) 2-hydroxyethyl methacrylate (HEMA) and a spatula tip of hydroquinone. The solution was stirred for two days at 90 °C. Thereafter, the solution was concentrated by distilling the solvent, precipitated in acetonitrile and stirred for 1 h in 2 mL water. The polymer was precipitated again in acetonitrile, isolated and dried in vacuum.

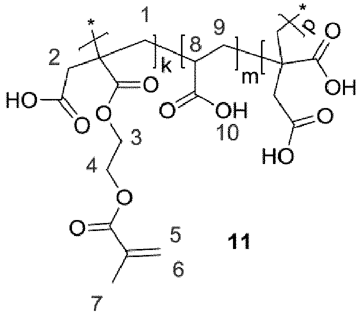

**Yield:** 75-80 % (conversion HEMA: 10%)
**FT-IR (Diamante):** ṽ_{max, br}[cm⁻¹]: 3209, 2955, 1716 (vᵥₐₗ. C=O), 1636 (vᵥₐₗ. -C=C), 1451, 1404, 1299, 1162 (-COO-R), 947, 815.
**¹H-NMR** (300 MHz, D₂O): δ (ppm)= 12.5 (br, 10), 6.2 (br, 5), 5.7 (br, 6), 4.4 (br, 3,4), 2.8 (br, 2), 2.4 (br, 1,9), 1.8 (br, 8), 1.3 (br, 7).

### Application example and comparative examples

The composition of the liquid 1 and of comparison liquids 2 to 4 are summarized in Table 1. For preparing the RMGI test specimens the liquid is always mixed with the named glass in a powder/liquid ratio of 2.8 /1.

The flexural strength of the glass ionomer composition basing on liquids of example 1 and of comparison example 2 and 4 are given in Table 1.

**Table 1: Composition of the liquid 1 and of comparison liquids 2 to 4 and flexural strength of the glass ionomer compositions**

| Liquid | HEMA-modified polyacid ^{a)} | Modified polyacid ^{b)} | PAA unmodified | AA | HEMA | BADEP | water | Initiator/ inhibitor | Example | Flexural strength |
|---|---|---|---|---|---|---|---|---|---|---|
| | wt% | | wt% | wt% | wt% | wt% | wt% | wt% | | MPa |
| 1 | 0.000 | 25.000 | 0.000 | 25.000 | 0.000 | 15.000 | 33.855 | 1.145 | Appl. Ex. 1 | 116 |
| 2 | 35.000 | 0.000 | 0.000 | 0.000 | 0.000 | 25.000 | 38.855 | 1.145 | Comparison example 2 | 61 |
| 3 | 35.000 | 0.000 | 0.000 | 0.000 | 25.000 | 0.000 | 38.855 | 1.145 | Comparison example 3 | 36 |
| 4 | 0.000 | 0.000 | 35.000 | 15.000 | 0.000 | 15.000 | 33.855 | 1.145 | Comparison example 4 | 63 |

### a) of comparative example 1, b) according example 2

- AA: Acrylic acid
- HEAA: 2-Hydroxyethylacryl amide
- HEMA: 2-Hydroxyethylacrylate
- BADEP: 1,3-Bis(acrylamido)-N,N'-diethylpropane

## Claims

1. Process for the preparation of a dental resin-modified glass ionomer composition comprising a polymerizable polyacidic polymer,wherein the process comprises the following steps:
(a) providing a linear polyacidic acrylic polymer having repeating units of following formula (I) 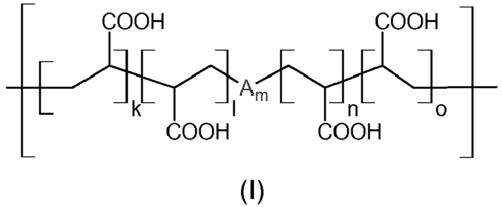 wherein
A , which may be the same or different, independently is selected from a group of the following formula (Ia) to (Ic): 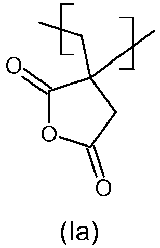 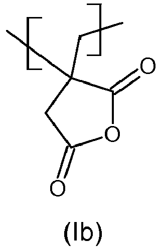 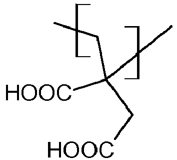 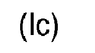 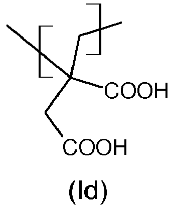 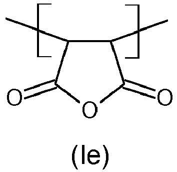 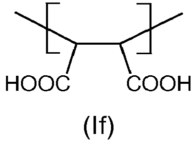
k, l, m, n and o are independently integers of at least 0,
k+l+m+n+o is at least 2; and
at least one of k, l, n, and o is at least 1;
wherein the polyacidic polymer has a weight average molecular weight of 10 to 300 kDa;
(b) reacting the linear polyacidic acrylic polymer with one or more polymerizable compounds of the following formula (II) in a solvent:
R-X (II)
wherein
X is selected from an amino group and an isocyanato group; and
R is an organic group having one or more polymerizable double bond;
for preparing a polymerizable linear polyacidic acrylic polymer having polymerizable pendant groups linked to the acrylic polymer backbone by amide groups; and
(c) combining the polymerizable linear polyacidic acrylic polymer having polymerizable pendant groups linked to the acrylic polymer backbone by amide groups with a reactive dental glass capable of reacting with the polyacidic acrylic polymer in a cement reaction.

2. The process according to claim 1, wherein the linear polyacidic acrylic polymer is polyacrylic acid or a copolymer of acrylic acid and itaconic anhydride.

3. The process according to any one of claim 1 or 2, wherein R in formula (II) is a moiety of the following formula (III): 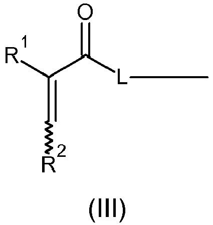 wherein
R¹ represents a hydrogen atom, a carboxylic acid group or a C₁₋₃ alkyl group;
R² represents a hydrogen atom, a carboxylic acid group or a C₁₋₃ alkyl group;
L represents a divalent organic linker group.

4. The process according to any one of claims 1 to 3, wherein L is a group
-YL'-,
wherein
Y represents O or NH, and
L' represents a divalent organic group.

5. The process according to any one of the preceding claims, wherein X is an amino group and the carboxylic acid groups of the linear polyacidic acrylic polymer are activated with a coupling agent prior to the reaction with the polymerizable compounds of the formula (II).

6. The process according to any one of the preceding claims, wherein the coupling agent is a carbodiimide.

7. The process according to claim 6, wherein the carbodiimide is N,N'-dicyclohexylcarbodiimide (DCC), N-(3-Dimethylaminopropyl)-N'-ethylcarbonate (EDC), or N,N'-diisopropylcarbodiimide (DIC).

8. The process according to any one of the preceding claims, wherein 0.02 to 0.5 eq. of the one or more polymerizable compounds of the formula (II) are reacted based on the total number of carboxylic acid groups of the linear polyacidic acrylic polymer.

9. The process according to any one of the preceding claims, wherein the solvent is selected from the group of dimethylformamide (DMF), acetonitrile and carbon tetrachloride.

10. The process according to any one of the preceding claims, wherein the reaction between the linear polyacidic acrylic polymer having repeating units of the formula (I) and the one or more polymerizable compound of the formula (II) is carried out at a temperature of from 20 to 100 °C for 1 to 60 hours.

11. The process according to any one of the preceding claims, wherein the linear polyacidic acrylic polymer having polymerizable pendant groups linked to the acrylic polymer backbone by amide groups has repeating units of the following formula (IV) 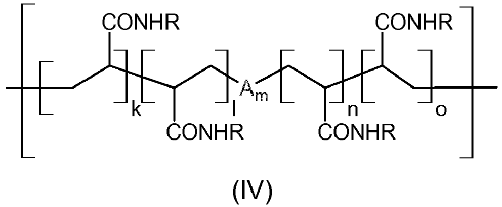 wherein
R is as defined in claim 1,
k, l, m, n, and o are independently integers of at least 0,
k+l+m +n+o is at least 2; and
at least one of k, l, n, and o is at least 1;
A which may be the same or different, independently represent a group selected from groups of the following formula (IVc), (IVd), and (IVf): 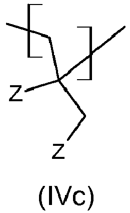 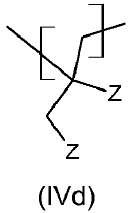 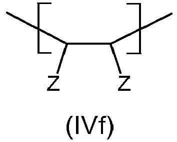 wherein Z is COOH or CONHR' (wherein R' is as defined in claim 1), and
wherein the polyacidic polymer has a weight average molecular weight of 12 to 400 kDa.

12. The process according to any one of the preceding claims, wherein the process further comprises adding an initiator system and optionally one or more polymerizable monomers to the dental resin-modified glass ionomer composition.

13. The dental resin-modified glass ionomer composition obtainable according to any one of claim 1 to 12.

14. The dental resin-modified glass ionomer composition according to claim 13, which is a two-pack composition.

15. The dental resin-modified glass ionomer composition according to any one of claims 13 to 15, for use as a temporary or final restoration of a hard dental tissue.
